# EUROPÄISCHE PATENTSCHRIFT

(11) **EP 1 529 206 B1**
(45) Veröffentlichungstag und Bekanntmachung des Hinweises auf die Patenterteilung: **07.05.2008**
(21) Anmeldenummer: 03793705.9
(22) Anmeldetag: 07.08.2003
(51) Int. Cl.: G01N 15/05, G01N 33/574

(54) **VERFAHREN ZUM IMMUNZYTOLOGISCHEN ODER MOLEKULAREN NACHWEIS VON DISSEMINIERTEN TUMORZELLEN AUS EINER KÖRPERFLÜSSIGKEIT UND DAZU GEEIGNETER KIT**
METHOD FOR THE IMMUNOCYTOLOGICAL OR MOLECULAR DETECTION OF DISSEMINATED TUMOR CELLS IN A BODY FLUID AND KIT THAT IS SUITABLE THEREFOR
PROCEDE DE DETECTION IMMUNOCYTOLOGIQUE OU MOLECULAIRE DE CELLULES TUMORALES DISSEMINEES DANS UN LIQUIDE CORPOREL ET KIT ASSOCIE

(30) Priorität: 14.08.2002 DE 10237296
(43) Veröffentlichungstag der Anmeldung: 11.05.2005
(73) Patentinhaber: HEXAL Gentech ForschungsGmbH, 83607 Holzkirchen (DE)
(72) Erfinder: DAHM, Michael, W., 81677 München (DE)
(74) Vertreter: Keller, Günter
(86) Internationale Anmeldenummer: PCT/EP2003/008796
(87) Internationale Veröffentlichungsnummer: WO 2004/023110

(56) Entgegenhaltungen:
- WO-A-00/46585
- WO-A-96/17080
- WO-A-97/21488
- US-A- 5 840 502
- GOETZ ALEXANDER W ET AL: "Enrichment of disseminated breast cancer cells from human peripheral blood by advanced density gradient centrifugation" PROCEEDINGS OF THE AMERICAN ASSOCIATION FOR CANCER RESEARCH ANNUAL MEETING, Bd. 43, März 2002 (2002-03), Seite 733, XP002261837 & 93RD ANNUAL MEETING OF THE AMERICAN ASSOCIATION FOR CANCER RESEARCH; SAN FRANCISCO, CALIFORNIA, USA; APRIL 06-10, 2002 ISSN: 0197-016X
- BISCHOFF J ET AL: "Detection of disseminated tumor cells in bone marrow and peripheral blood of patients with metastatic breast cancer" ANNUAL MEETING OF THE AMERICAN SOCIETY OF CLINICAL ONCOLOGY, Mai 2002 (2002-05), XP002261838 ORLANDO FL, USA
- ROSENBERG R ET AL: "Comparison of two density gradient centrifugation systems for the enrichment of disseminated tumor cells in blood." CYTOMETRY, Bd. 49, Nr. 4, 1. Dezember 2002 (2002-12-01), Seiten 150-158, XP002261834 & ISSN: 0196-4763
- HEXAL GENTECH FORSCHUNGS-GMBH: ONCOQUICK-TM GEBRAUCHSANLEITUNG, [Online] XP002261835 Gefunden im Internet: <URL:http://www.hexal-gentech.de/products/ man_d.pdf>
- GÖTZ A: "Mit OncoQuick den Metastasen auf der Spur" BIOFORUM, Bd. 24, 2001, Seite 394, XP002261836 GIT VERLAG GMBH, DARMSTADT

## Beschreibung

Die Erfindung betrifft ein Verfahren zum Nachweis von Tumorzellen aus einer Körperflüssigkeit sowie ein dazu geeignetes Kit.

Nahezu alle soliden malignen Tumore haben das Potential zur Metastasenbildung. Der Prozeß der Metastasierung beinhaltet die Aussaat maligner Zellen als Mikrometastasen, zumeist auf dem Blut- bzw. Lymphweg in entfernte Organe und die Ausbildung autonomer Tochtergeschwülste. Das Ausmaß der Filiarisierung bestimmt die Prognose eines Tumorleidens.

Die Ansprüche von Tumorvorsorge- oder -nachsorgeprogrammen liegen in der Früherkennung von Primärtumoren bzw. Rezidiven oder von Metastasen noch bevor diese klinisch manifest werden. Dieses Ziel kann bislang mit den verfügbaren apparativen Techniken nicht zufrieden stellend erfüllt werden. Durch den Nachweis von zirkulierenden bzw. disseminierten Tumorzellen z.B. im peripheren Blut könnte frühzeitig, d.h. noch vor dem Auftreten eines klinisch bemerkten Tumors, eine möglicherweise kurative Immunmodulations- oder Polychemotherapie eingeleitet werden. Die Quantifizierung der Tumorzellen im peripheren Blut vor und nach der Therapie stellt dabei eine wichtige Kontrolle dar.

Diesbezüglich offenbart die WO 00/46585 ein Verfahren zur Anreicherung von Tumorzellen durch eine Dichtezentrifugation. Als analytische Methode wurde hierbei anschließend das RT-PCR-Verfahren eingesetzt. Der epithelzellspezifische Marker CK20 (Zytokeratin 20) konnte hierbei nicht nachgewiesen werden.

Des Weiteren offenbart der Artikel von Götz et al. (Proceedings of the American Association for Cancer Research Annual Meeting, Bd. 43, März 2002, Seite 733) die Verwendung von OncoQuick® als Zentrifugationsgefäß zur Anreicherung von Tumorzellen. Als Analysemethode wird hierbei der Nachweis von DAPI-markierten Tumorzellen beschrieben.

Ein erfolgversprechender Ansatz zum Nachweis und zur Quantifizierung von disseminierten Tumorzellen besteht in der Bestimmung von epithelialen Markern wie Zytokeratinen in einer Körperflüssigkeit. Beispielsweise beschreiben Racila et al. in Proc Natl Acad Sci USA (1998) 95: 4589 einen Assay, mit dem disseminierte epitheliale Tumorzellen mittels monoklonaler Antikörper gegen Zytokeratin im peripheren Blut nachgewiesen werden können. Weiterhin existieren bereits kommerzielle molekulare Kitsysteme, die erlauben in einer Probe zum Beispiel die Zytokeratin 20-Expression mittels PCR zu bestimmen (Roche LightCycler-CK20 Quantification Kit).

Zytokeratine gehören zu der Familie von filamentösen Proteinen, die am Aufbau des Zytoskeletts von epithelialen Zellen beteiligt sind (Moll et al. in Cell (1982) 31: 11). Nachdem epitheliale Zellen im peripheren Blut normalerweise nicht vorkommen, wird postuliert, daß eine Zytokeratinexpression im Blut ein Surrogatmarker für das Vorhandensein von disseminierten Tumorzellen ist.

Im peripheren Blut können jedoch neben Tumorzellen auch andere Blutzellen wie polymorphkernige Leukozyten, speziell Granulozyten, epitheliale Marker wie Zytokeratine exprimieren (Jung et al. Br J Cancer (1999) 81: 870). Aufgrund dieser Tatsache muß vor dem Nachweis eines epithelialen Markers, wie z.B. einer Zytokeratinexpression als Surrogatmarker für disseminierte Tumorzellen im Blut eine Trennung dieser den epithelialen Marker exprimierenden, wie z.B. Zytokeratin exprimierenden Nicht-Tumorzellen von den einen epithelialen Marker, wie z.B. Zytokeratin exprimierenden Tumorzellen vorgenommen werden.

In den 60er- und 70er-Jahren, als Methoden wie beispielsweise FACS oder MACS noch nicht zur Verfügung standen, wurden Tumorzellen von hämatopoetischen Zellen aufgrund ihrer unterschiedlichen Dichte getrennt (J.A. Fleming et al., J. clin. Path. (1967), 20, 145). Entsprechend dieser Daten besitzen Tumorzellen eine spezifische Dichte von 1,040-1,080, während Erythrozyten ein höhere und Lymphozyten eine ähnliche Dichte aufweisen. Polymorphkernige Leukozyten weisen hingegen eine spezifische Dichte im Bereich von 1,060-1,080 auf und überschneiden sich somit mit der spezifischen Dichte von Tumorzellen. Eine vollständige Abtrennung der ebenfalls Zytokeratin-positiven Granulozyten von den Tumorzellen über deren Dichteunterschiede sollte somit nicht möglich sein. So zeigte auch die Verwendung einer Standardlösung zur Isolierung von Lymphozyten, wie z.B. Histoprep^{®} (Sigma) mit einer Dichte von 1,077 g/ml, daß Granulozyten von gesunden Blutspendern mit einer Dichte von bis zu 1,077 g/ml Zytokeratin-positiv sind (Jung et al. Br J Cancer (1999) 81: 870).

Eine Aufgabe der vorliegenden Erfindung besteht somit darin, ein vorteilhaftes Verfahren zum Nachweis von Tumorzellen aus einer Körperflüssigkeit zur Verfügung zu stellen.

Es wurde nun überraschend gefunden, daß durch OncoQuick®, ein in WO 99/40221 und WO 00/46585 beschriebenes Verfahren, Zytokeratin-exprimierende Nicht-Tumorzellen von Zytokeratin-exprimierenden Tumorzellen abgetrennt werden können. OncoQuick® (Greiner Bio One, Frickenhausen, Germany) verwendet als diskontinuierlichen Gradienten ein Zellseparationsmedium, das eine höhere Dichte als die zu untersuchende Körperflüssigkeit aufweist und mit der Körperflüssigkeit überschichtet wird. Durch Zentrifugation werden die Zellen ihrer spezifischen Zelldichte nach aufgetrennt und können in einzelnen Fraktionen abgenommen werden. Überraschend wurde gefunden, daß durch den spezifischen Dichtegrad des Zellseparationsmediums Zytokeratin-positive von Zytokeratin-negativen Blut-Zellen getrennt werden können, wobei sich die angereicherten Tumorzellen nach der Zentrifugation in der gleichen Fraktion befinden wie die Zytokeratin-negativen Blut-Zellen, so daß eine anschließend nachgewiesene Zytokeratinexpression in dieser Fraktion zweifelsfrei auf vorhandene Tumorzellen zurückgeführt werden kann.

Die vorliegende Erfindung betrifft daher ein Verfahren zum Nachweis von disseminierten Tumorzellen aus einer Körperflüssigkeit, wie in Anspruch 1 angegeben.

Eine Expression eines epithelialen Markers kann dann als Surrogat für die Präsenz von disseminierten Tumorzellen gewertet werden.

Bei der Körperflüssigkeit, aus der Zytokeratin-positive Tumorzellen angereichert werden sollen, kann es sich um menschliche oder tierische Körperflüssigkeiten oder um eine Dispersion von Zellgewebe handeln. Hierbei handelt es sich beispielsweise um Blut, insbesondere peripheres Blut wie venöses oder arterielles Blut, Pfortaderblut oder Blut aus einem zentralen venösen Katheter (ZVK), Lymphe, Urin, Exsudate, Transudate, Spinalflüssigkeit, Samenflüssigkeit, Speichel, Flüssigkeiten aus natürlichen oder unnatürlichen Körperhöhlen, Knochenmark und dispergiertes Körpergewebe. Bei den Flüssigkeiten aus natürlichen Körperhöhlen kann es sich beispielsweise um seröse Flüssigkeiten wie Peritoneal- und Pleuraflüssigkeiten handeln, bei den Flüssigkeiten aus unnatürlichen Körperhöhlen kann es sich beispielsweise um Flüssigkeiten aus Zysten handeln.

Bevorzugte Körperflüssigkeiten sind Blut, Knochenmark, Lymphe, seröse Flüssigkeiten aus Körperhöhlen sowie Urin, wobei Blut und Urin besonders bevorzugt sind. Urin eignet sich insbesondere zur Anreicherung von Zellen von Blasentumoren.

Die bevorzugteste Körperflüssigkeit ist jedoch peripheres Blut, das vorteilhaft in einer gerinnungshemménden Substanz abgenommen und vor dem Überschichten des Zellseparationsmediums mit einem Verdünnungsmittel verdünnt wird. Als gerinnungshemmende Substanzen können beispielsweise EDTA oder Citrat bzw. Heparin oder CPD (Citrat, Phosphat, Dextrose) oder vergleichbare Substanzen eingesetzt werden. Als peripheres Blut eignet sich venöses oder arterielles Blut.

Bevorzugte Tumorzellen sind Tumorzellen, die von einem soliden Tumor stammen, insbesondere von Tumoren epithelialen Ursprungs und besonders bevorzugt Tumorzellen, die ein Zytokeratin exprimieren. Epitheliale Tumoren sind beispielsweise Karzinome, wie Karzinome der Brust, der Prostata oder gastrointestinale Tumoren, ohne sich jedoch darauf zu beschränken.

Die zu untersuchende Körperflüssigkeit wird, wie in WO 99/40221 und WO 00/46585 beschrieben, entsprechend gängiger Standardprotokolle entnommen bzw. gesammelt. In Abhängigkeit von der Art der Körperflüssigkeit wird diese dann entweder zunächst mit einem Verdünnungsmittel, bevorzugt einem Puffer, verdünnt oder direkt unverdünnt in einem verschließbaren Zentrifugationsgefäß über das Zellseparationsmedium geschichtet.

Alternativ kann die Körperflüssigkeit zuvor bei beispielsweise 1.000 x g für ca. 10 Minuten zentrifugiert und nach Resuspension der Zellen in einem Puffer über das Zellseparationsmedium geschichtet werden. Der bevorzugt verwendete Puffer ist Dulbecco PBS.

Die Zentrifugation wird vorteilhaft bei ca. 500 bis 2.000 x g, bevorzugt bei ca. 1.000 x g über ca. 10 bis 30 Minuten, bevorzugt über 20-30 Minuten durchgeführt. Die Temperatur während der Zentrifugation beträgt vorzugsweise ca. 4°C. Dies bewirkt, daß die katalytische Aktivität von Proteasen, DNAsen bzw. RNAsen möglichst gering gehalten wird.

Als Zellseparationsmedium läßt sich im Prinzip jede geeignete Flüssigkeit gewünschter Dichte verwenden. Die Dichte liegt im Bereich von 1,055 bis 1,065 g/ml, vorzugsweise im Bereich von 1,059 bis 1,062 g/ml und ist am bevorzugtesten 1,060 ± 0,0005 g/ml. In einer besonderen Ausführungsform ist die Dichte kleiner als 1,065 g/ml, also 1,055 bis < 1,065 g/ml. Das Zellseparationsmedium sollte nicht mit der Körperflüssigkeit oder den darin enthaltenen Zellen reagieren. Vorteilhaft kann beispielsweise Ficoll® oder Percoll bzw. ein Percoll- oder Ficoll®-ähnliches Medium verwendet werden, wobei die Lösungen jeweils nach Anweisungen des Herstellers auf die gewünschte Dichte gebracht werden. Vorteilhafterweise wird die Dichte des Zellseparationsmediums mit Hilfe eines Dichtemeßgeräts (DMA 4500, Anton Paar, Österreich) bei der entsprechenden Arbeitstemperatur von 4°C eingestellt. Die in WO 00/46585 beschriebene Vorgehensweise zur Herstellung einer Lösung gewünschter Dichte ist durch Bezugnahme Teil der Offenbarung dieser Anmeldung. Das Zellseparationsmedium kann einen Farbstoff enthalten, der das Zellseparationsmedium von der darüberliegenden Körperflüssigkeit farblich unterscheidbar macht und dadurch die Lokalisation der Interphase vereinfacht.

Um einen möglichst einfachen Ablauf der Arbeiten zu sichern, kann, wie in WO 99/40221 und WO 00/46585 beschrieben, in einer bevorzugten Ausführungsform des erfindungsgemäßen Verfahrens die Zentrifugation in einem verschließbaren Gefäß durchgeführt werden, das durch eine Barriere, einen Filter oder ein Sieb, nachfolgend poröse Barriere oder Barriere genannt, in ein oberes und ein unteres Kompartiment geteilt ist, wobei das Zellseparationsmedium im unteren Kompartiment vorgelegt und die Körperflüssigkeit in das obere Kompartiment eingebracht wird.

Die Barriere hat vorzugsweise eine poröse Beschaffenheit, die es erlaubt, daß bei der Zentrifugation Flüssigkeiten sowie die korpuskulären Bestandteile des Blutes, wie die Zellen des roten und weißen Blutsystems, welche eine höhere Dichte als das vorgelegte Zellseparationsmedium haben, die poröse Barriere ungehindert passieren können. Dies hat zur Folge, daß das Zellseparationsmedium während der Zentrifugation durch die poröse Membran in das obere Kompartiment gedrängt wird und die Tumorzellen und Thrombozyten, welche eine geringere Dichte als das vorgelegte Zellseparationsmedium haben, auf einer Ebene oberhalb der Barriere zu liegen kommen.

Die poröse Barriere kann aus jedem geeigneten Material bestehen. Beispielsweise eignen sich Kunststoff, Metall, Keramik oder eine Mischung oder spezielle Legierung dieser Stoffe. Es kann aber auch jedes andere natürliche oder künstliche, geeignete Material eingesetzt werden. In einer bevorzugten Ausführungsform besteht die poröse Barriere aus einem hydrophoben Material oder ist mit einem hydrophoben Material beschichtet.

In einem Zentrifugationsgefäß kann alternativ zur Barriere, wie in WO 99/40221 und WO 00/46585 beschrieben, auch eine Klappe eingesetzt werden, die das Zentrifugationsgefäß analog zur Barriere in ein oberes und ein unteres Kompartiment unterteilt.

Diese Klappe hat eine Beschaffenheit, die es erlaubt, daß sie vor und nach der Zentrifugation dicht verschlossen ist und nur während der Zentrifugation durch die Zentrifugationskraft geöffnet wird. Während der Zentrifugation treffen bei geöffneter Klappe die Flüssigkeiten des unteren und des oberen Kompartiments zusammen. Dies hat zur Folge, daß die Zellen des roten und weißen Blutsystems, welche eine höhere Dichte als das vorgelegte Zellseparationsmedium haben, in das untere Kompartiment gelangen und das Zellseparationsmedium in das obere Kompartiment drängen. Dies hat den Effekt, daß die Tumorzellen, welche eine geringere Dichte als das vorgelegte Zellseparationsmedium haben, auf einer Ebene oberhalb der Klappe zu liegen kommen.

Die poröse Barriere, der Filter, das Sieb oder die Klappe kann eine Dicke von 0,5 - 10 mm, vorzugsweise von 1 - 5 mm aufweisen. Die poröse Barriere, der Filter, das Sieb oder die Klappe kann eine Porengröße von 20 - 100 µm, vorzugsweise 20 - 30 µm aufweisen.

Mit Hilfe der porösen Barriere oder Klappe kann die zu untersuchende Körperflüssigkeit in das Zentrifugationsgefäß gefüllt werden, ohne daß sie sich mit dem darunterliegenden Zellseparationsmedium vermischt und somit die Anreicherung beeinträchtigen oder unmöglich machen kann.

Nach der Zentrifugation befinden sich die in der Körperflüssigkeit vorhandenen Tumorzellen in der Interphase des oberen Kompartiments des Zentrifugationsgefäßes. Die Interphase wird zwischen dem Trennmedium und der Restflüssigkeit einer Körperflüssigkeit oder einer Zellsuspension ausgebildet und enthält die angereicherten Tumorzellen. Bei der Restflüssigkeit handelt es sich beispielsweise bei der Verwendung von Blut als Körperflüssigkeit um Plasma, ein Plasma/PBS-, bzw. Plasma/Puffer-Gemisch, das die Proteine des Plasmas enthält. Die Flüssigkeit oberhalb der Interphase kann dann zu etwa 80% vorsichtig abgesaugt und verworfen werden.

Der verbleibende Überstand oberhalb der Barriere oder Klappe, in dem sich die Tumorzellen befinden, kann anschließend gesammelt und beispielsweise in ein frisches Zentrifugationsgefäß (vorzugsweise aus gegebenenfalls silikonisiertem Kunststoff und mit der gleichen Volumenkapazität wie das zuvor verwendete Zentrifugationsgefäß) überführt werden. Die poröse Barriere bzw. die geschlossene Klappe verhindert bei der Entnahme des verbleibenden Überstands ein Vermischen der Zellen des oberen und des unteren Kompartiments.

Vorteilhaft wird anschließend das obere Kompartiment des Zentrifugationsgefäßes beispielsweise, wie in WO 99/40221 und WO 00/46585 beschrieben, zweimal mit einem Puffer nachgewaschen und dieser wird ebenfalls in das frische Zentrifugationsgefäß überführt. Nach einer weiteren Zentrifugation beispielsweise bei 1.000 x g über ca. 10 Minuten bei einer Temperatur von ca. 4°C können die gesammelten Zellen dann den Tumorzellnachweismethoden zugeführt werden. Als Waschpuffer eignet sich z.B. Dulbecco's PBS (3,9 mM EDTA; pH 8,0; ohne Ca/Mg). Weitere Waschpuffer sind in WO 99/40221 und WO 00/46585 offenbart.

Bei Verwendung eines Zentrifugationsgefäßes mit poröser Barriere bzw. Klappe wird, wie in WO 99/40221 und WO 00/46585 beschrieben, nach Entnahme der überstehenden Restflüssigkeit oberhalb der Interphase jedoch bevorzugt nicht nur die Interphase, sondern die gesamte verbliebene Flüssigkeit oberhalb der porösen Barriere entnommen, nicht weil sich zwischen Interphase und poröser Barriere bzw. Klappe noch weitere Zellen befinden, sondern weil durch die Abnahme die im Zellring enthaltenen Zellen leicht durchmischt werden können. Um keine Zellen aus dem oberen Kompartiment zu verlieren wird dieses vorteilhaft noch zweimal mit einem Puffer (z.B. PBS mit 0,1% - 10% BSA) gewaschen.

Mit dem erfindungsgemäßen Verfahren können disseminierte Tumorzellen und insbesondere disseminierte Tumorzellen von epithelialen Tumoren aufgrund ihrer unterschiedlichen Dichte vollständig von den korpuskulären Bestandteilen von Körperflüssigkeiten wie z.B. den Zellen des roten und weißen Blutsystems getrennt, konzentriert und z.B. die Zytokeratinexpression der angereicherten Tumorzellen mittels immunzytologischer bzw. molekularbiologischer Tumorzellnachweisverfahren nachgewiesen werden. Im Sinne dieser Anmeldung exprimiert eine Zelle einen bestimmten epithelialen Marker, wie z.B. ein bestimmtes Zytokeratin, wenn sie das Protein des epithelialen Markers, wie z.B. ein Zytokeratin-Protein, oder die dafür kodierende mRNA enthält. Bevorzugte epitheliale Marker sind Zytokeratine. Zytokeratine, die erfindungsgemäß nachgewiesen werden können, sind Zytokeratine 1-20, bevorzugt 1, 4-8, 10-11, 13-20, besonders bevorzugt eine Kombination aus den Zytokeratinen 1, 4-8, 10-11, 13-20. Die bevorzugteste Kombination für den immunzytochemischen Nachweis sind Antikörper für Zytokeratin 8, 18, 19 und 20. Die bevorzugten Zytokeratine für den molekularen Nachweis sind Zytokeratin 18, 19 und 20, am bevorzugtesten ist Zytokeratin 20. Beispiele weiterer epithelialer Marker sind das Prostate Specific Antigen (PSA, Israeli et al., 1994, Cancer Res 54, 6306-6310), die Human Melanoma Antigen- (MAGE-) kodierende Genfamilie (De Plaen et al., 1994, Immunogenetics 40, 360-369, Hasegawa et al., 1998, Pathol Lab Med 122, 551-554), Mamma-spezifische Antigene wie MAS-385, SB-6 (Ross, et al., 1993, Blood 82, 2605-2610), MUC-1 (Brugger et al., 1999, J Clin Oncol 17, 1535-1544) und GA733-2 (Zhong et al., 1999, Tumordiagn. u. Ther. 20, 39-44), Adhäsionsmoleküle wie TFS-2, EPCAM (Racila et al., 1998, Proc Natl Acad Sci U S A 95, 4589-4594), oder CEACAM1 (Thies et al., 2002, J Clin Oncol 20, 2530-2536), Rezeptormoleküle wie Östrogen-Progesteron-Rezeptoren (Bitran et al., 1992, Dis Mon 38, 213-260), das Carcinoembrionic Antigen (CEA, Liefers et al., 1998, N Engl J Med 339, 223-228), das PRL-3 Protein, eine Tyrosin Phosphatase (Saha et al. 2001, Science 294, 1343-1346) oder Maspin, ein Protein aus der Familie der Serpine (Sabbatini et al., 2000, J Clin Oncol 18, 1914-1920) ohne sich darauf zu beschränken.

Die bekannten Methoden zum Nachweis einer Expression von epithelialen Markern, insbesondere der Zytokeratin-Expression umfassen das gesamte Spektrum der gängigen Diagnostik. Beispiele hierfür sind die mikroskopischen, immunzytologischen/immunzytochemischen, biochemischen und/oder molekularbiologischen Verfahren. Beispielsweise kann die Expression von epithelialen Markern, insbesondere die Zytokeratin-Expression nach der Anreicherung direkt oder nach Zellkultur und Expansion der Tumorzellen durch morphologische, immunzytologische/immunzytochemische, biochemische und/oder molekularbiologische Verfahren nachgewiesen werden. Dabei können Zellbestandteile epithelialer Marker, insbesondere Zytokeratin-spezifische Zellbestandteile nachgewiesen werden, z.B. das Protein eines epithelialen Markers, wie z.B. das Zytokeratin-Protein selbst, spezifische Abschnitte von Chromosomen bis hin zu Nukleinsäuresequenzen, wie DNA, RNA und cDNA.

Erfindungsgemäß umfaßt die Bestimmung, ob die angereicherten Zellen einen epithelialen Marker, ausgewählt aus Zytokeratin 1 bis 20, exprimieren, daß man mRNA aus den angereicherten Zellen revers transkribiert und eine PCR mit wenigstens einem für den epithelialen Marker spezifischen Primer, z.B. einem Zytokeratin-spezifischen Primer durchführt. In einem anderen, nicht erfindungsgemäßen Verfahren, bringt man die angereicherten Zellen mit einem monoklonalen Antikörper in Kontakt, der spezifisch für ein epitheliales Antigen, wie z.B. ein bestimmtes Zytokeratin ist, und weist anschließend an die Zellen gebundenen Antikörper nach. Diese Verfahren können in Einzel- oder Kombinationsanalyse durchgeführt werden. In anderen bekannten Verfahren werden Antikörper verwendet, die wie im Falle des murinen monoklonalen Antikörpers A45-B/B3 (Micromet, Deutschland) ein gemeinsames Epitop von verschiedenen Zytokeratinen (8, 18 und 19) erkennen. Es können auch Antikörper kombiniert werden, wie z.B. der Antikörper A45-B/B3 zusammen mit einem Zytokeratin 20-Antikörper. Im Falle einer molekularen Analyse können z.B. PCR-Untersuchungen mit zwei spezifischen Primern bzw. Primerpaaren durchgeführt werden, die für zwei unterschiedliche epitheliale Marker spezifisch sind. Alternativ können die o.a. Untersuchungen auch mit einem anderen Antikörper bzw. mit einem anderen Primer bzw. Primerpaar, die für einen weiteren epithelialen Marker spezifisch sind, wiederholt werden. In einem anderen bekannten Verfahren werden "custom made" Mikroarrays verwendet, mit denen unterschiedliche epitheliale Marker in Kombination mit tumorspezifischen Markern wie z.B. der Telomerase, in einem Untersuchungsschritt nachgewiesen werden können.

Beispiele bekannter direkter Nachweisverfahren sind u.a. sämtliche Formen der Mikroskopie einschließlich der Färbung von Zellbestandteilen. Ein Beispiel direkter Färbung ist die Färbung durch spezifische Antikörper, die gegen Zytokeratine gerichtet sind und an denen Markierungssignale wie z.B. fluoreszierende Farbstoffe gekoppelt sind. Nachweisverfahren sind u.a. Durchflußzytometrie oder FACS (Fluorescence activated cell sorting), ELISA und Western Blotting. Weitere bekannte Verfahren zum Nachweis von epithelialen Markern, wie z.B. von Zytokeratinen sind u.a. die Detektionsverfahren von Nukleinsäuren mit Hilfe von markierten Sonden, z. B. FISH, in situ Hybridisierung, Northem, South-Western und Southern Blotting oder differential display sowie u.a. die Amplifikationsverfahren von Nukleinsäuren u.a. die PCR, RT-PCR, in situ RT-PCR, Real-Time PCT und NASBA. Dabei können Reagenzien zum Einsatz kommen, die mehrere epitheliale Marker, wie z.B. Zytokeratine nachweisen, also spezifisch für eine Gruppe von epithelialen Markern, wie z.B. Zytokeratinen sind. Es können aber auch Reagenzien verwendet werden, die nur einen bestimmten epithelialen Marker, wie z.B. nur ein bestimmtes Zytokeratin spezifisch nachweisen, z.B. ein monoklonaler anti-CK-20-Antikörper oder CK-20-spezifische Primer zum spezifischen RT-PCR-Nachweis von CK-20. Anti-Zytokeratin-Antikörper sind kommerziell erhältlich oder können nach an sich bekannten Verfahren hergestellt werden. Primer können aus den cDNA-Sequenzen der verschiedenen Zytokeratine abgeleitet werden. Die cDNA-Sequenz von CK-20 ist in der NCBI Datenbank http://www.ncbi.nlm.nih.gov unter der Accession No. BC031559 (Homo sapiens, cytokeratin 20, clone MGC:35423, IMAGE:5189289, mRNA, complete cds) hinterlegt.

Die angeschlossenen Nachweisverfahren können auf folgenden Gebieten zum Einsatz kommen:

Der Nachweis der Expression von epithelialen Markern, wie z.B. den der Zytokeratin-Expression in den angereicherten Zellen kann direkt als Tumormarker eingesetzt werden. In Staginguntersuchungen kann die Anzahl der nachgewiesenen disseminierten Tumorzellen mit dem klinischen Bild korreliert und ein individuelles Tumorstaging festgelegt werden. Nach Entfernung des Primärtumors kann der Patient regelmäßigen Rezidivkontrollen unterzogen und bei positivem Befund sofort behandelt werden. Weitere Anwendungsmöglichkeiten sind der Nachweis von residualen Tumorzellen im Knochenmark von Patienten, die sich einer Hochdosis-Strahlentherapie unterziehen müssen oder von disseminierten Tumorzellen im Rahmen von neuen Therapieansätzen, sowie ex-vivo und in-vivo Gentherapieansätzen.

Einen besonderen Stellenwert wird dem Verfahren im Rahmen von Tumorvorsorgeuntersuchungen beigemessen, da mit einer erheblich früheren Diagnose und bei sofortiger Therapie mit längeren Überlebensraten zu rechnen ist. Weitere Anwendungen liegen in der Tumorvakzineherstellung und der Gentherapie.

Die vorliegende Erfindung betrifft somit auch ein Verfahren zum Nachweis von Tumorzellen in einer Körperflüssigkeit, worin die Tumorzellen, wie vorstehend beschrieben, aus einer Körperflüssigkeit angereichert und bevorzugt gleichzeitig unerwünschte Zellen in einem hohen Maße abgereichert und disseminierte epitheliale Zytokeratin- Marker exprimierende Tumorzellen mittels molekularbiologischer Techniken nachgewiesen werden.

Ein weiterer Gegenstand der vorliegenden Erfindung ist ein Kit zur Anreicherung von disseminierten Tumorzellen aus einer Körperflüssigkeit, der zur Durchführung des erfindungsgemäßen Verfahrens geeignet ist. Hierzu umfaßt der Kit das in WO 99/40221 und WO 00/46585 offenbarte flüssige Zellseparationsmedium, das erfindungsgemäße Zentrifugations gefäß und Mittel zum Nachweis der Expression von wenigstens einem der epithelialen Zytokeratin 1-20- Markern, nach der Anreicherung direkt oder nach Zellekultur und Expansion der Tumorzellen, wobei das Mittel aus Zytokeratin-spezifischen Primers ausgewählt ist, sowie gegebenenfalls Waschpuffer in gegebenenfalls konzentrierter Form zum Waschen der angereicherten Zellen. Die bevorzugten Ausführungsformen der Bestandteile des Kits entsprechen den für das erfindungsgemäße Verfahren beschriebenen.

Um das routinemäßige Arbeiten mit dem Kit zu erleichtern, weist das Zentrifugationsgefäß eine poröse Barriere, einen Filter, ein Sieb oder Klappe auf, die das Zentrifugationsgefäß in ein oberes und ein unteres Kompartiment unterteilt. Die vorteilhaften Ausführungsformen des Zentrifugationsgefäßes entsprechen den oben für das erfindungsgemäße Verfahren beschriebenen.

Ebenfalls bekannte Verfahren zum Nachweis der aus einer Körperflüssigkeit angereicherten Tumorzellen, verwenden spezifische monoklonale Antikörper, mit denen epitheliale Marker exprimierende, wie z.B. Zytokeratin exprimierende Tumorzellen immunzytologisch nachgewiesen werden können. Bei den Antikörpern kann es sich solche handeln, die mehrere Zytokeratine erkennen, oder um solche, die nur ein Zytokeratin erkennen.

In Einer weiteren bevorzugten Ausführungsform der vorliegenden Erfindung zum Nachweis der aus einer Körperflüssigkeit angereicherten Tumorzellen, umfaßt der oben beschriebene Kit spezifische Primer mit denen epitheliale Zytokeratin-Marker exprimierende Tumorzellen mittels Reverse Transkriptase-Polymerase Kettenreaktion (RT-PCR) oder mittels Real Time- Polymerase Kettenreaktion (PCR) nachgewiesen werden können. Vorzugsweise handelt es sich um Primer, die nur eine cDNA eines epithelialen Markers, bzw. nur eine Zytokeratin-cDNA spezifisch amplifizieren, besonders bevorzugt um Primer die nur CK-18-, CK-19- und/oder CK-20-cDNA spezifisch amplifizieren, am bevorzugtesten um Primer, die nur CK-20-cDNA spezifisch amplifizieren.

Das erfindungsgemäße Verfahren bietet den Vorteil, daß epitheliale Marker-positive wie z.B. Zytokeratin-positive Nicht-Tumorzellen einfach und sicher von den anzureichernden Tumorzellen abgetrennt werden, so daß in anschließenden Nachweisverfahren keine falsch-positiven Ergebnisse durch epitheliale Marker-positive wie z.B. Zytokeratin-positive Nicht-Tumorzellen erhalten werden. So können Nicht-Tumorzellen, die wenigstens eines der Zytokeratine 1-20 exprimieren, von Tumorzellen, die wenigstens eines der Zytokeratine 1-20 exprimieren, abgetrennt werden. Insbesondere können CK-20-positive Nicht-Tumorzellen von CK-20-positiven Tumorzellen abgetrennt werden. Darüber hinaus sind nur wenige Arbeitsschritte für die Anreicherung und Isolierung von Tumorzellen aus Körperflüssigkeiten notwendig, so daß dadurch die Prozessierung von größeren Mengen von Probenmaterial möglich wird. Die Kosten für die notwendigen Materialien sind beispielsweise gegenüber der Verwendung spezifischer Antikörper und der anschließenden Trennung mittels geeigneter Apparaturen signifikant geringer.

Der unwahrscheinliche Fall, daß ein falsch positives Ergebnis erhalten wird, kann zusätzlich dadurch ausgeschlossen werden, daß ein bestimmter Wert festgesetzt wird, ab dem ein Ergebnis als positiv gewertet wird ("cut off'). Zum Beispiel kann ein Signal nach RT-PCR insbesondere nach Real-Time PCR mit einer gespikten Probe mit 2-10 Zellen/ml (z.B. mit 2, 4, 6, 8 oder 10 Zellen/ml) als Mindestsignal für einen positiven Nachweis festgelegt werden. Die Real-Time PCR bietet die Möglichkeit, mittels einer Quantifikation Software (Roche) die Kopiennummer des Amplifikats zu bestimmen. Liegt der Wert des Amplifikats über einer zuvor ermittelten Mindestkopiennummer, kann das Ergebnis als positiv gewertet werden. Ähnlich kann mit custom made Mikroarrays verfahren werden, indem die Intensität des ermittelten Signals über einem zuvor bestimmten Intensitätswert liegen muß.

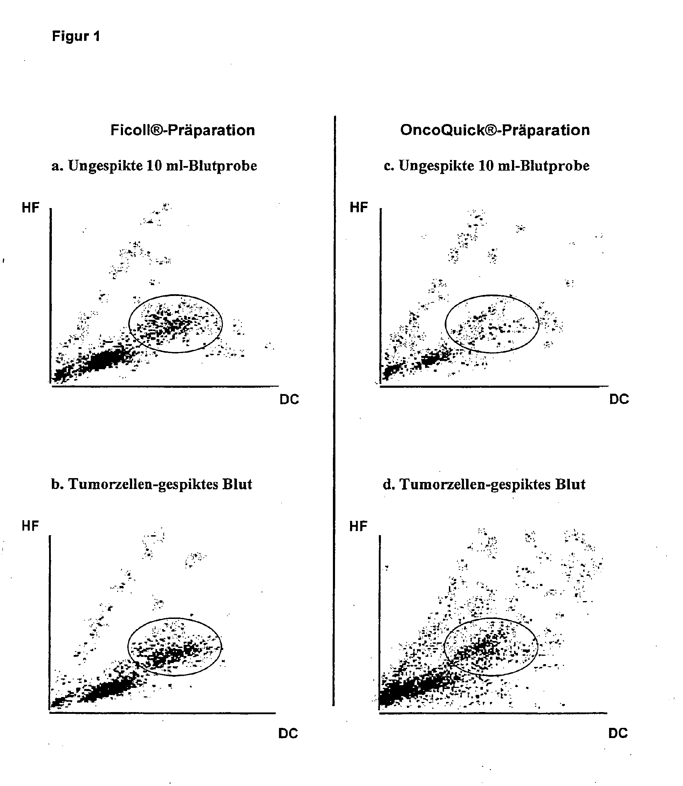

Figur 1 zeigt eine hämatologische Analyse (Sysmex SE-9000) von 10 ml peripherem Blut gesunder Probanden, das mit Ficoll® (a,b) oder OncoQuick® (c,d) aufgereinigt wurde. a) und c) sind ungespikte Proben, b) und d) sind mit Tumorzellen gespikte Proben. Die ungespikten Blutproben enthielten 1.8 x 10⁷ Blutzellen nach Ficoll®- (a) und 9.5 x 10⁴ nach OncoQuick®-Anreicherung (c). Nachdem zu den Proben 2.7 x 10⁶ Tumorzellen gespiked worden waren, bildet die Tumorzellfraktion (hauptsächlich in der von der Ellipse gekennzeichneten Region) die Hauptfraktion nach der OncoQuick®-Anreicherung (d), während die Tumorzellen, nach Ficoll®-Anreicherung (b) mit einer großen Anzahl MNCs angereichert werden. HF= "high frequency", DC= "direct current").

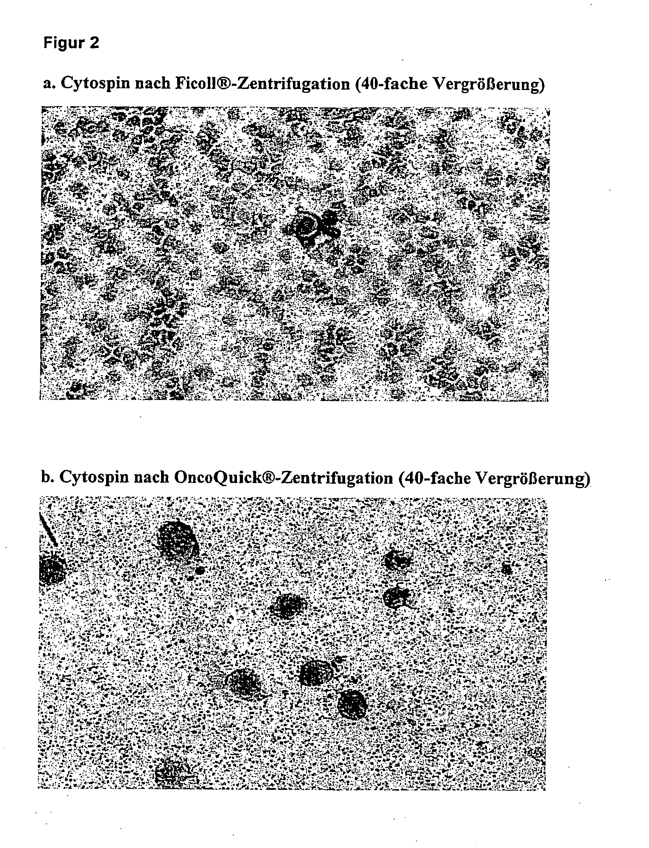

Figur 2 zeigt als nicht unter den Schutzumfang fallendes Beispiel den immunzytologischen Nachweis mit dem Panzytokeratin-Antikörper A45-B/B3 von 10 ml peripherem Blut gesunder Probanden, mit 100 gespiketen Tumorzellen nach einer Anreicherung mit Ficoll® (a) oder OncoQuick® (b). Nach einer Anreicherung mit OncoQuick® wurde die gesamte angereicherte Zellfraktion mit einer erhöhten Tumorzelldichte auf 1-2 Objektträger zentrifugiert, im Vergleich zu 50 Objektträgern nach einer Ficoll®-Anreicherung.

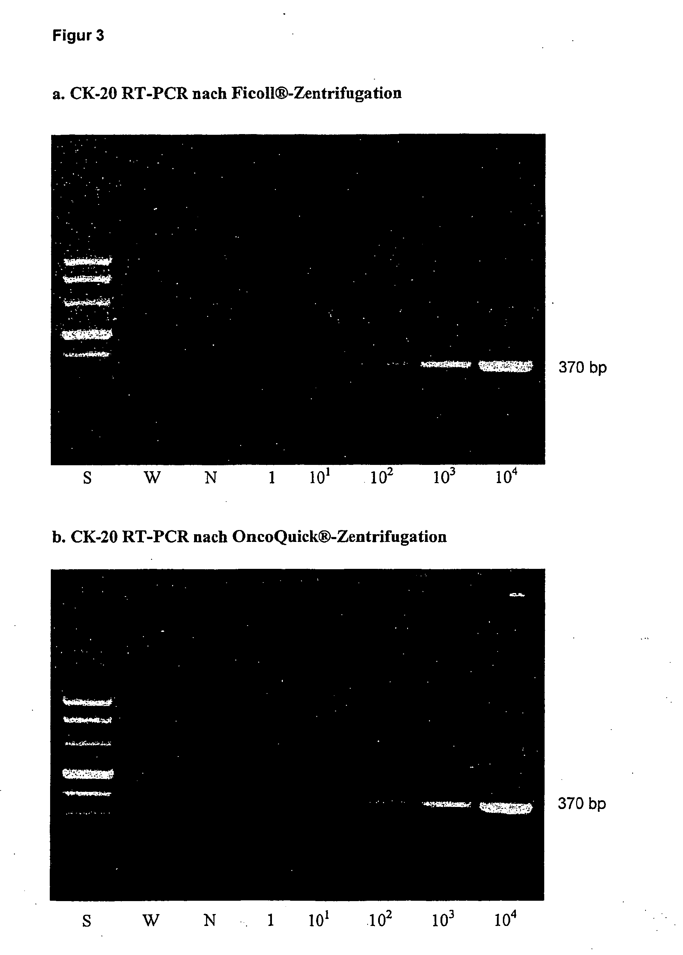

Figur 3 zeigt eine CK-20 RT-PCR von Blutproben, die mit 1 bis 10⁴ Tumorzellen gespiked wurden, nach einer Anreicherung mit Ficoll® (a) oder OncoQuick® (b). Während mit OncoQuick® 10 Tumorzellen in 30ml Blut nachgewiesen werden können, sind Präparationen mit Ficoll® auf eine Menge von nur 10ml Blut limitiert. Wasser (W), NegativKontrollen (N) und DNA Längen Standard VII (Roche, Deutschland) (S).

Die nachfolgenden Beispiele erläutern die Erfindung näher.

### Beispiel 1

Dieser Versuch wurde durchgeführt, um festzustellen, ob CK20 mRNA-exprimierende Nicht-Tumorzellen durch OncoQuick® erfolgreich abgereichert werden können.

OncoQuick® ist ein kommerziell erhältliches Zellseparationssystem, das folgende Merkmale aufweist: Es umfaßt ein 50 ml Zentrifugationsgefäß, das durch eine poröse Barriere in 2 Kompartimente unterteilt ist. Das untere Kompartiment enthält das Zellseparationsmedium mit einer Dichte im erfindungsgemäßen Bereich. In das obere Kompartiment kann die zu untersuchende Körperflüssigkeit eingefüllt werden.

Der Versuch wurde an einer Gruppe von 70 gesunden Probanden durchgeführt und die Auswertung mit der sensibelsten Amplifikationsmethode, der Real Time PCR vorgenommen. Von insgesamt 70 gesunden Probanden wurde jeweils eine Gesamtmenge von 30ml peripheren Blutes abgenommen und in 2 gleiche Aliquote von 15ml aufgeteilt. Ein Aliquot wurde jeweils mit Ficoll® (Leucosep® Greiner Bio One), das andere Aliquot mit OncoQuick® (Greiner Bio One) nach Angaben des Herstellers aufgereinigt. 50 ml Leucosep®-Zentrifugationsgefäße enthalten wie OncoQuick® eine poröse Barriere, die das Röhrchen in ein oberes und in ein unteres Kompartiment unterteilt. Im unteren Kompartiment sind 15 ml Ficoll® vorgelegt. 10 ml Blut wurde in das Zentrifugationsgefäß eingebracht und bei 1000g für 10 Minuten bei Raumtemperatur zentrifugiert. Analog wurden 10 ml Blut in ein vorgekühltes OncoQuick®-Zentrifugationsgefäß eingebracht und bei 1600g für 20 Minuten bei 4°C zentrifugiert. Das gesamte obere Kompartiment wurde entnommen und in ein frisches 50ml Zentrifugationsgefäß überführt. Die gesammelten Zellen wurden einmal mit PBS (Böhringer, Deutschland) bei 500g für 15 Minuten (Ficoll®) und mit dem OncoQuick®-Waschpuffer bei 200g für 12 Minuten (OncoQuick®) bei 4°C gewaschen. Die gewaschenen Zellen wurden dann gesammelt und die totale RNA mit dem High Pure RNA Isolations Kit (Roche, Deutschland) nach Angaben des Herstellers extrahiert. Die RNA wurde dann in 70 µl eluiert. 10 µl der eluierten RNA wurde dann in eine 20 µl Reverse Transkriptase Reaktion des LightCycler-CK20 Quantification Kit (Roche, Deutschland) überführt und anschließend 5 µl in die Real-Time PCR nach Angaben des Herstellers eingesetzt. Bei der Auswertung der Ergebnisse zeigte sich, daß in den mit Ficoll® angereicherten Proben 8 individuelle Proben CK-20 positiv waren. Im Vergleich dazu waren die mit OncoQuick® aufgereinigten Proben negativ.

### Beispiel 2

### 1. Material und Methoden

### Dichtegradientenzentrifugation der Blutproben

Für alle Experimente wurde Tumor-freien Patienten mit EDTA-beschichteten 10 ml Monovetten (Sarstedt, Deutschland) peripheres venöses Blut abgenommen, das innerhalb von zwei Stunden nach Blutabnahme verarbeitet wurde. Alle Blutproben wurden mit Zustimmung der Patienten gemäß Institutsrichtlinien und nach IRB-Genehmigung gewonnen. Alle Experimente wurden zehnmal durchgeführt und Mittelwerte wurden aus den Ergebnissen berechnet. Mononukleäre Zellen (MNCs) wurden aus Gesamtblut durch Ficoll®- (Biochrom Deutschland) und durch OncoQuick®- (Greiner BioOne Deutschland) Dichtegradientenzentrifugation abgetrennt.

### OncoQuick® - Dichtegradientenzentrifugation

Vorgekühlte 50 ml Zentrifugenröhrchen, die 15 ml Separationsmedium unterhalb einer porösen Barriere, die ein oberes und ein unteres Kompartiment trennt, enthielten, wurden mit 10 bis 30 ml Blut gefüllt und bei 1600 g für 20 min bei 4°C in einem Außschwingrotor (Hettich, Deutschland) zentrifugiert. Das Gesamtvolumen des oberen Kompartiments einschließlich der Interphase mononukleärer Zellen wurde in ein frisches Zentrifugenröhrchen gegossen. Die poröse Barriere verhinderte jegliche Kontamination der separierten MNCs mit der Fraktion an pelletierten Blutzellen. Danach wurden die Zellen mit Waschpuffer bei 200 g für 12 Minuten bei 4°C zentrifugiert, der Überstand wurde abgesaugt. Die pelletierten Zellen wurden zur weiteren Untersuchung verwendet.

### Ficoll® Dichtegradientenzentrifugation

50 ml Zentrifugenröhrchen mit einer porösen Filterscheibe bei 15 ml (Leucosep^{®}, Greiner BioOne Deutschland) enthaltend 15 ml Ficoll® wurden mit 10 ml Blut gefüllt und bei 10.000 g für 10 min bei Raumtemperatur zentrifugiert. Um die MNCs zu gewinnen, wurde das gesamte Volumen des oberen Kompartiments des Leucosep^{®}-Röhrchens in ein frisches 50 ml Zentrifugenröhrchen gegossen. Die gewonnenen Zellen wurden mit PBS (Böhringer, Deutschland) bei 500 g für 15 min bei 4°C gewaschen. Der Überstand wurde abgesaugt und für den weiteren Nachweis disseminierter Tumorzellen verwendet. Die niedrigen g-Werte, die für die Waschschritte eingesetzt wurden (500 g für Ficoll® und 200 g für OncoQuick®) wurden gewählt, um die Zellintegrität für die immunzytologischen Untersuchungen zu gewährleisten.

### Abreicherung von Blutzellen nach Dichtegradientenzentrifugation

Zur Quantifizierung der abgetrennten Zellen durch die beiden Dichtegradientenzentrifugationsmethoden wurde von 10 Tumor-freien und gesunden Probanden 30 ml Blut abgenommen und in drei 10 ml Aliquote aufgeteilt. Für jeden Patienten wurde ein Aliquot mit Ficoll® und ein Aliquot mit OncoQuick® vorbereitet. Das dritte Aliquot blieb unbearbeitet. Die durch die beiden Zentrifugationsmethoden abgetrennten Zellen und die unbearbeitete Gesamtblutprobe wurden mit der hämatologischen Analysevorrichtung Sysmex SE-9000 (Sysmex Deutschland), die klinisch zur Blutzelldifferenzierung eingesetzt wird, analysiert. "Scattergraphen" wurden durch Widerstandsmessung mit Gleichstrom und hochfrequentem Wechselstrom erstellt, wodurch die dreidimensionale Verteilung von Zellgröße und interner Zelldichte gezeigt wird.

### Tumorzellgewinnung nach Dichtezentrifugation

Um den Anteil an gewonnenen Tumorzellen nach Präparation von Gesamtblut mit Ficoll® und OncoQuick® zu bestimmen, wurden "Spiking-Experimente" mit der colorektalen Karzinomzelllinie HT-29 durchgeführt. Aus zehn tumorfreien Probanden wurde jeweils 40 ml Blut abgenommen und in vier 10 ml Aliquote aufgeteilt. Zwei Aliquote eines jeden Probanden wurden mit 2,7 x 10⁶ oder 2,7 x 10⁵ Tumorzellen gespiked während die übrigen zwei Aliquote nicht manipuliert wurden. Ein Paar aus ungespiketer und gespiketer Blutprobe wurde mit Ficoll® bearbeitet, das andere Paar mit OncoQuick®, und mit der hämatologischen Analysevorrichtung untersucht. Die Differenz in der Gesamtzahl an MNCs zwischen den gespiketen und ungespiketen Proben wurde den gespiketen Tumorzellen zugerechnet. Ein Vergleich der Anzahl an gespiketen Tumorzellen mit dieser berechneten Anzahl an gewonnenen Tumorzellen nach Ficoll®- oder OncoQuick®-Behandlung diente als ein Maß für den Anteil an gewonnenen Tumorzellen mit der jeweiligen Methode.

### Nachweis disseminierter Tumorzellen

Der Tumorzellnachweis wurde durch Immuncytochemie (ICC) und Reverse Transkriptase Polymerase Kettenreaktion (RT-PCR) durchgeführt. Um die Nachweisrate gespiketer Tumorzellen in Blut, das mit entweder Ficoll® oder OncoQuick® behandelt worden ist, zu bestimmen, wurden 10 ml Blutproben von Tumor-freien Probanden mit einer ansteigenden Anzahl an Tumorzellen (0, 10, 100 und 1000 Tumorzellen für RT-PCR Nachweis und 100 und 1000 Tumorzellen für ICC Nachweis) gespiked.

*Immuncytochemie* als nicht unter den Schutzumfang fallendes Beispiel Die Gesamtzahl an abgetrennten MNCs mit und ohne gespikete Tumorzellen wurde durch Mikroskopie mit einer Neubauer Zellkammer bestimmt. Maximal 200.000 Zellen wurden auf jedes Deckgläschen zentrifugiert. Die so durch Zytozentrifugation erhaltenen Präparate wurden mit Aceton 10 min bei Raumtemperatur fixiert, luftgetrocknet, und dann mit antikörperfreiem humanem Serum 25 min vorinkubiert, um unspezifische Antikörperbindung zu blocken. Zytokeratin-positive Zellen wurden durch den Maus Anticytokeratin monoklonalen Antikörper (MAb) A45-B/B3 (Micromet, Deutschland) identifiziert, der ein gemeinsames Epitop auf verschiedenen Cytokeratin-Proteinen einschließlich Cytokeratin 8, 18 und 19 nachweist, und mit dem monoklonalen Antikörper CK-20 (Dako Deutschland), der gegen Cytokeratin 20 (CK 20) gerichtet ist. Nach einer 45-minütigen Inkubation mit dem Primärantikörper wurde die Reaktion mit der Alkalische Phosphatase-Antialkalische Phosphatase (APAAP) - Technik entwickelt. Die Anzahl an Zytokeratin-positiven Zellen wurde mikroskopisch von zwei Personen unabhängig voneinander bestimmt.

### Cytokeratin-20 RT-PCR

Die Isolierung von Gesamt-RNA aus den Zellpellets, die durch Ficoll®- oder OncoQuick®-Behandlungen erhalten wurden, wurde mit dem "High Pure RNA Isolation Kit" (Roche, Deutschland) durchgeführt. Die Amplifizierung von CK-20 mRNA (Moll et al., Differentiation 1993; 53: 75 - 93) wurde in einer einstufigen RT-PCR mit dem "Titan One Tube RT-PCR Kit" (Roche, Deutschland) durchgeführt. Die PCR wurde in einem Reaktionsvolumen von 50 µl einschließlich höchstens 29,5 µl Matrizen RNA für OncoQuick®-Proben und höchstens 10 µl Matrizen-RNA für Ficoll®-Proben durchgeführt, um RNA-Überladung zu vermeiden. Mit einem Set CK-20 spezifischer Primer, die von Funaki et al., Br. J. Cancer 1998; 77: 1327 - 1332 beschrieben wurden, wurde eine RT-PCR Amplifikation für alle Proben in einem Varius-V Thermocycler (Landgraf, Deutschland) durchgeführt, wie in einer früheren Studie beschrieben (Rosenberg et al., J Clin Oncol 2002; 20: 1049 - 1055). Eine effiziente Amplifikation wurde durchgehend von allen Proben erhalten und mit einer β2-Microglobulin RT-PCR kontrolliert. Die PCR Produkte wurden auf einem 2% Agarosegel aufgetrennt. Für geeignete Negativkontrollen wurde die Matrizen-RNA durch Nuklease-freies Wasser ersetzt. Als Positivkontrolle wurde extrahierte Gesamt-RNA aus der kolorektalen Karzinomzelllinie HT-29 analysiert.

### Spezifität von CK-20 RT-PCR in Kombination mit OncoQuick®

Um die Spezifität von CK-20 RT-PCR für den Nachweis von Tumorzellen zu bestimmen, wurden Blutproben von 30 Tumor-freien Patienten untersucht, die keine Anzeichen einer Krebserkrankung zeigten. 30 Patienten mit Knochenbrüchen wurde jeweils 30 ml Blut abgenommen. Die Gesamtblutmenge wurde in zwei Hälften geteilt zur weiteren Dichtegradientenzentrifugation mit OncoQuick® und Ficoll®. CK-20 RT-PCR wurde nach RNA Extraktion aus allen Blutproben durchgeführt.

Zusätzlich wurden 15 ml Blutproben von 5 Tumor-freien Patienten auf CK-20 Expression in der Zellfraktion oberhalb der porösen Barriere und in der Zellfraktion unterhalb der porösen Barriere nach OncoQuick® oder Ficoll® Dichtegradientenzentrifugation untersucht. Die Zellfraktionen oberhalb und unterhalb der porösen Barriere der Blutproben wurden nach Dichtegradientenzentrifugation getrennt, Gesamt-RNA wurde extrahiert und durch CK-20 RT-PCR und Real-time CK-20 LightCycler RT-PCR (Soong et al., Clin Cancer Res 2001; 7: 3423 - 3429) zur Quantifizierung von CK-20 - Expression untersucht. Zusätzlich wurde in der Zellfraktion unterhalb der porösen Barriere, die die Erythrozyten enthält, eine Lyse der Erythrozyten durchgeführt.

### Klinische Evaluierung

Um den klinischen Nutzen von OncoQuick® zu validieren, wurden schließlich 37 Patienten, die zum Staging von Magen-Darm-Krebs untersucht wurden, untersucht. Von jedem Patienten wurden zwei Proben von 20 ml peripherem Blut präoperativ gewonnen. Alle Blutproben wurden mit dem Dichtegradienten OncoQuick® zentrifugiert. Der Nachweis zirkulierender Tumorzellen wurde durch Zytokeratin CK-20 RT-PCR durchgeführt. Die Nachweisraten wurden mit der Präsenz von Fernmetastasen verglichen und statistisch durch den "chi-square-Test" bei einem Signifikanzwert von p≤0,05 analysiert.

### 2. Ergebnisse

### Quantifizierung und Charakterisierung der angereicherten Zellfraktion

### Abreicherung von Blutzellen nach Dichtegradientenzentrifugation

Die Charakterisierung der verschiedenen Blutzellfraktionen von 10 ml unbehandeltem Gesamtblut durch die hämatologische Analysevorrichtung ergab eine mittlere Zellzahl von 6,0 x 10⁷ weißen Blutkörperchen (WBC), 4,6 x 10¹⁰ Roten Blutkörperchen (RBC) und 2,3 x 10⁹ Blutplättchen. Die WBC-Differenzierung ist in Tabelle 1 gezeigt.

Tabelle 1 zeigt den Vergleich der Zellzahl aus der angereicherten Interphase von 10 ml peripherem Blut gesunder Probanden nach einer Anreicherung mit Ficoll® und OncoQuick®. Das Verhältnis von Zellzahl vor und nach Dichtegradientenanreicherung bildet den Abreicherungsfaktor.

| | | 10 ml Blut | Ficoll-Präparation | | OncoQuick-Präparation | |
|---|---|---|---|---|---|---|
| | | n=10 | n=10 | | n=10 | |
| | | Zellzahl | Zellzahl | Abreicherung | Zellzahl | Abreicherung |
| Rote Blutkörperchen | | 4,6 × 10¹⁰ | 8,0 × 10⁷ | 575-fach | 3,6 × 10⁶ | 12778-fach |
| | ± SD | ± 3,5 × 10⁹ | ± 2,8 × 10⁷ | | ± 1,7 × 10⁶ | |
| Blutplättchen | | 2,3 × 10⁹ | 4,1 × 10⁸ | 5,6-fach | 1,6 × 10⁸ | 14,4-fach |
| | ± SD | ± 8,6 × 10⁸ | ± 1,7 × 10⁸ | | ± 5,2 × 10⁷ | |
| Weiße Blutkörperchen | | 6,0 × 10⁷ | 1,6 × 10⁷ | 3,8-fach | 9,5 × 10⁴ | 632-fach |
| | ± SD | ± 1,6 × 10⁷ | ± 5,2 × 10⁶ | | ± 1,5 × 10⁴ | |
| Lymphozyten | | 1,9 × 10⁷ | 1,1 × 10⁷ | 1,7-fach | 2,7 × 10⁴ | 704-fach |
| | ± SD | ± 1,1 × 10⁷ | ± 4,9 × 10⁶ | | ± 1,7 × 10⁴ | |
| Monozyten | | 5,0 × 10⁶ | 3,5 × 10⁶ | 1,4-fach | 1,0 × 10⁴ | 500-fach |
| | ± SD | ± 1,5 × 10⁶ | ± 3,4 × 10⁵ | | ± 3,0 × 10³ | |
| Neutrophile | | 3,5 × 10⁷ | 8,7 × 10⁵ | 40-fach | 5,4 × 10⁴ | 648-fach |
| | ± SD | ± 7,0 × 10⁶ | ± 4,0 × 10⁵ | | ± 2,5 × 10⁴ | |
| Eosinophile | | 9,4 × 10⁵ | 4,9 × 10⁴ | 19-fach | 2,0 × 10³ | 470-fach |
| | ± SD | ± 6,2 × 10⁵ | ± 8,7 × 10⁴ | | ± 2,8 × 10³ | |
| Basophile | | 3,2 × 10⁵ | 2,0 × 10⁵ | 1,6-fach | 4,0 × 10³ | 80-fach |
| | ± SD | ± 3,3 × 10⁵ | ± 1,8 × 10⁵ | | ± 3,2 × 10³ | |

Die Interphase, die aus 10 ml Blut nach Ficoll®-Behandlung wiedergefunden wurde, bestand aus 1,6 x 10⁷ WBC, 8,0 x 10⁷ RBC und 4,1 x 10⁸ Blutplättchen. Die gesammelte Interphase nach OncoQuick®-Behandlung von 10 ml Blut bestand aus 9,5 x 10⁴ WBC, 3,6 x 10⁶ RBC und 1,6 x 10⁸ Blutplättchen. Durch Ficoll®-Zentrifugation wurde die Anzahl an WBC um Faktor 4 reduziert, die Anzahl an RBC um Faktor 575 und die der Blutplättchen um Faktor 6 verglichen mit den unbehandelten Gesamtblutfraktionen. Zum Vergleich dazu führt die OncoQuick®-Zentrifugation zu einer stärkeren Reduktion der gesammelten Zellen mit einem Abreicherungsfaktor von 632 für WBC, 12.778 für RBC und 14 für Blutplättchen, verglichen mit den Fraktionen des unbehandelten Gesamtbluts.

### Tumorzellgewinnung nach Dichtezentrifugation

Die Wiederfindung von 2,7 x 10⁵ und 2,7 x 10⁶ gespiketen Tumorzellen war vergleichbar für beide Zentrifugationsmethoden (Bereich 70% bis 90%) mit einem Mittelwert von 84% für Ficoll® und 87% für OncoQuick®, analysiert mit der hämatologischen Analysevorrichtung (Figur 1).

### Nachweis disseminierter Tumorzellen

*lmmuncytochemie* als nicht unter den Schutzumfang fallendes Beispiel Mikroskopische Quantifizierung der abgetrennten MNCs mit einer Neubauer Zellkammer ergab eine mittlere Menge von 2 x 10⁵ Zellen nach OncoQuick®-Zentrifugation und 1 x 10⁷ Zellen nach Ficoll®-Zentrifugation. Zur weiteren ICC Evaluierung wurde eine maximale Zellzahl von 2 x 10⁵ Zellen pro Deckgläschen zentrifugiert. Dementsprechend konnten alle MNCs, die durch OncoQuick® abgetrennt wurden, auf ein bis zwei Deckgläschen zentrifugiert werden, wohingegen ungefähr 50 "Cytospins" zur Untersuchung aller MNCs, die durch Ficoll® abgetrennt wurden, notwendig waren. In "spiking"-Experimenten mit 100 und 1.000 Tumorzellen in 10 ml Gesamtblut wurde eine mittlere Wiederfindungsquote von 42% (Bereich 25-70%) durch ICC bestimmt unabhängig vom verwendeten Anreicherungssystem. Nach Ficoll®-Zentrifugation wurden lediglich 1-2% der gespiketen Tumorzellen pro Deckglas nachgewiesen, wohingegen OncoQuick® den Nachweis von 25-70% der gespiketen Tumorzellen pro Deckglas erlaubte (Figur 2).

Beim Vergleich der zwei monoklonalen Antikörper, die zur Identifizierung der Tumorzellen verwendet wurden, wurde eine höhere Sensitivität für A45-B/B3 verglichen mit CK-20 in Blut gefunden. Um die Nachweisgrenze für ICC zu bestimmen, wurden "spiking"-Experimente mit 10 und 100 gespiketen Tumorzellen in 30 ml Blut entsprechend den RT-PCR Experimenten durchgeführt. Mit dem monoklonalen Antikörper A45-B/B3 wurde in einer Reihe von fünf wiederholten Experimenten zweimal eine von 10 gespiketen Tumorzellen nach OncoQuick®-Zentrifugation gefunden, während keine von 10 gespiketen Tumorzellen mit dem monoklonalen Antikörper CK-20 oder nach Ficoll®-Zentrifugation identifiziert wurden.

### Cytokeratin-20 RT-PCR

Die extrahierten mittleren Gesamt-RNA-Ausbeuten waren 20µg nach Ficoll®- und 1µg nach OncoQuick®-Dichtegradientenzentrifugation von jeweils 10 ml Blut. Die CK-20 RT-PCR Reaktion, die in diesem Beispiel verwendet wurde, war auf eine maximale Menge von 2 µg Gesamt-RNA-Ausbeute oder höchstens 30 µl RNA-Extrakt begrenzt. Daher konnte nur 10% der RNA Ausbeute nach Ficoll®-Behandlung in einer einzelnen PCR Reaktion analysiert werden, was der Analyse von lediglich ungefähr 1 ml Gesamtblut entspricht. Zur PCR Analyse von OncoQuick®-Präparationen konnte ungefähr 50% der erhaltenen RNA-Ausbeute in einer einzelnen PCR Reaktion analysiert werden.

Verdünnungsreihen mit der Colorektal-Krebszelllinie HT-29 zur Sensitivitätsüberprüfung von CK-20 RT-PCR ergab eine Nachweisgrenze von 10 gespiketen Tumorzellen in 10 ml Gesamtblut für Ficoll® Dichtegradientenzentrifugation (Figur 3). Entsprechend der überlegenen Abreicherung von Blutzellen durch OncoQuick®-Zentrifugation und die nachfolgende geringere Menge an Blutzell-RNA konnten die Blutvolumina auf bis zu 30 ml für OncoQuick®-Präparationen und RT-PCR-Analyse erhöht werden mit einer Nachweisgrenze von 10 gespiketen Tumorzellen in 30 ml Blut (Figur 3). Zusammenfassend kann festgestellt werden, daß Tumorzell-RNA in einer höheren Konzentration mit weniger kontaminierender Blutzell-RNA nach Blutpräparation mit OncoQuick® erhalten wurde, verglichen mit Ficoll®.

### Spezifität von CK-20 RT-PCR nach OncoQuick®-Dichtegradientenzentrifugation

Dreißig Tumor-freie Blutproben wurden auf "falsch-positive" CK-20 Expression aufgrund von illegitimer Transkription oder Pseudogenen untersucht. Alle Proben, die durch OncoQuick®-Zentrifugation abgereichert wurden, waren negativ in der RT-PCR für CK-20 (0%), wohingegen 3 von 30 Proben (10%), die durch Ficoll®-Zentrifugation abgereichert wurden, positiv waren, obwohl diese Patienten nicht Krebs hatten.

Zur weiteren Untersuchung wurden die Zellen unterhalb und oberhalb der porösen Schicht von 5 Tumor-freien Blutproben nach OncoQuick®-Zentrifugation analysiert. Während die mononukleäre Zellfraktion der Interphase oberhalb der porösen Barriere in allen 5 Tumor-freien Blutproben nach OncoQuick®-Zentrifugation keine CK-20 Expression zeigte, exprimierte die Zellfraktion unterhalb der porösen Barriere CK-20 in allen 5 Proben. Die Untersuchung von 10 ml Blut, das mit 100 HT29 Kolon Krebszellen gespiked wurde, zeigte CK-20 Expression in der Zellfraktion oberhalb und unterhalb der porösen Barriere. Zusätzliche Quantifizierung der CK-20 Expression mit einer quantitativen Lightcycler CK-20 RT-PCR, um zu bestimmen ob eine erhöhte CK-20 Expression in der Fraktion unterhalb der porösen Barriere nach Tumorzell-"spiking" gefunden wird, ergab eine mittlere Anzahl von CK-20-Kopien von 3.000 bis 8.000 in der gespiketen oder in der ungespiketen Zellfraktion. Die Interphase (die Zellfraktion oberhalb der porösen Barriere) der Tumorzell-gespiketen Blutprobe enthielt eine CK-20-Kopienanzahl von 380.000, während keine CK-20 Expression in der ungespiketen Zellfraktion oberhalb der porösen Barriere gefunden wurde. Diese Daten zeigen, daß nach OncoQuick®-Zentrifugation keine CK-20 exprimierenden Tumorzellen unterhalb der porösen Barriere sind.

### Klinische Anwendung

In peripherem Blut wurde CK-20 mRNA nach OncoQuick®-DichtegradientenZentrifugation in 11 von 37 Patienten gefunden (30%). 26 Patienten (70%) waren negativ in beiden Blutproben. 6 von 22 Patienten (27%) mit oberem Magen-Darm-Krebs exprimierten CK-20 mRNA in Proben von peripherem Blut und Patienten mit unterem Magen-Darm Krebs zeigten eine CK-20 mRNA Expression in 5 von 15 Patienten (33%). Ein Vergleich mit histopathologischen Daten zeigte, daß 6 von 29 Patienten (21 %) ohne klinische Evidenz für Fernmetastasen (pM0) CK-20 Expression zeigten, während 5 von 8 Patienten (63%) mit Fernmetastasen (pM1) CK-20 mRNA exprimierten (p<0,02) (Tabelle 2).

Tabelle 2 zeigt den Nachweis von CK-20 mRNA im peripheren Blut von 37 Patienten mit gastrointestinalen Karzinomen, nach Aufreinigung mit OncoQuick®. Tumorpatienten mit Fernmetastasen exprimieren wesentlich mehr CK-20 mRNA (63%) als Tumorpatienten ohne Anzeichen von Fernmetastasen (21%) (p≤ 0.02; "chi-square-Test").

| | Alle Patienten | | Fernmetastasen | | Keine Metastasen | |
|---|---|---|---|---|---|---|
| | | | pM1 | | pM0 | |
| | n | % | n | % | n | % |
| Alle Patienten | 37 | 100% | 8 | 22% | 29 | 78% |
| CK-20 positiv | 11 | 30% | 5 | 63% | 6 | 21 % |
| CK-20 negativ | 26 | 70% | 3 | 37% | 23 | 79% |

### Beispiel 3 nicht unter den Schutzumfang fallend-

### 1. Patienten und Methoden

### Patienten

Die Untersuchungsgruppe bestand aus 42 Patientinnen mit metastasierendem Brustkrebs, die zwischen November 2000 und Februar 2001 in der gynäkologischen Abteilung des Klinikums Bad Trissl behandelt wurden. Alle Frauen waren postmenopausal und erhielten hormonelle Behandlung, Chemotherapie oder chemoendokrine Therapie und Bisphosphonate. 13 Patientinnen hatten maligne Knochenläsionen als einzigen Metastasenort.

### Knochenmarksaspiration und Immunzytochemie

Eine Standard-Knochenmarkspunktion wurde bei allen 13 Patientinnen, die maligne Knochenmetastase als einzigen Metastasenort hatten, nach Zustimmungserklärung durchgeführt. Die Punktion an beiden Spina iliaca anterior superior wurde unter örtlicher Betäubung durchgeführt. 5 ml Knochenmark wurden erhalten, und beide Proben von jeder Patientin wurden vor der weiteren Bearbeitung gepoolt. Mononukleäre Zellen wurden durch Dichtegradientenzentrifugation mit Ficoll isoliert. Die Anzahl der mononukleären Zellen wurde durch Zytozentrifugation auf Objektträger zentrifugiert. Die Zytospins wurden fixiert und dann mit dem Panzyokeratin monoklonalen Antikörper A45-B/B3 (Micromet, Deutschland) gefärbt. Zur Visualisierung der Antikörperbindung wurde die APAAP-Technik benutzt (Cordell et al., 1984, J. Histochem. Cytochem.: 32, 219-229). Für jede Patientin wurden fünf Objektträger enthaltend insgesamt 2 x 10⁶ mononukleäre Zellen von zwei unabhängigen Beobachtern untersucht.

### Proben von peripherem Blut und Immunzytochemie

Zusätzlich wurden von allen 42 Patientinnen 20 ml Blutproben von der peripheren Ellenbogenvene abgenommen. Diese Patientinnen hatten Anzeichen von viszeralen und partiellen Knochenmetastasen. Die Blutproben von jeder Patientin wurden mit einem optimierten Dichtegradientensystem OncoQuick (Greiner BioOne, Deutschland) bearbeitet. Mononukleäre Zellen wurden vom Gesamtblut durch OncoQuick-Dichtegradientenzentrifugation isoliert und durch Zytozentrifugation auf Objektträger zentrifugiert. Epitheliale Zellen wurden mit dem monoklonalen Antikörper A45-B/B3 und der APAAP-Technik gefärbt. Aufgrund der verbesserten Depletion mononukleärer Zellen bei der Verwendung von OncoQuick im Vergleich zu Ficoll wurden alle mononukleären Zellen auf durchschnittlich zwei Objektträger enthaltend bis zu 2 x 10⁶ mononukleäre Zellen zentrifugiert, die auch mikroskopisch von zwei unabhängigen Beobachtern untersucht wurden.

### 2. Ergebnisse

### Zytokeratin-positive Zellen in Knochenmark und peripherem Blut

Zytokeratin-positive Zellen wurden bei 21 Patientinnen im peripheren Blut und bei 3 Patientinnen im Knochenmark nachgewiesen. Die Anzahl nachgewiesener Zellen in peripherem Blut lag zwischen 1 und mehr als 20 pro 10⁶ Zellen. 1 Zytokeratin-positive Zelle pro 1 x 10⁶ mononukleären Interphase-Zellen wurde in den zwei positiven Knochenmarksproben gefunden. 7 Patientinnen hatten negative Befunde im Knochenmark und peripheren Blut. 3 Patientinnen waren Zytokeratin-positiv im peripheren Blut und negativ im Knochenmark, wohingegen in 2 Patientinnen Zytokeratin-positive Zellnachweise im Knochenmark, nicht aber in peripherem Blut gefunden wurden.

### Zytokeratin-positive Zellen und Metastasenort

9 von 14 Patientinnen mit Lebermetastasen, aber nur 5 von 16 Patientinnen mit Knochenmetastasen und 3 von 7 Frauen mit pulmonalem Metastasenort hatten positive Befunde im peripheren Blut. Diese Daten zeigen einen signifikanten Unterschied in der Nachweisrate Zytokeratin-positiver Zellen in peripherem Blut von Brustkrebspatientinnen mit hepatischer Tumorstreuung im Vergleich mit anderen metastatischen Manifestationen. Keine klare Korrelation konnte zwischen der Anzahl nachgewiesener Zellen und dem Ort der Metastasen gefunden werden.

### Zytokeratin-positive Zellen und Überlebensrate

15 der 42 untersuchten Patientinnen starben innerhalb eines nachfolgenden Zeitraums von 9 Monaten. Alle verstarben aufgrund krebsbezogener Ursachen. 11 dieser Patientinnen hatten positive Befunde in peripherem Blut. 8 dieser Frauen hatten Lebermetastasen. Diese Daten zeigen, daß die Gegenwart Zytokeratin-positiver Zellen in peripherem Blut mit dem klinischen Folgezustand assoziiert ist.

### Schlußfolgerung

Dichtegradientenzentrifugation mit OncoQuick in Verbindung mit immunzytochemischer Färbung erlaubt die Identifizierung isolierter Tumorzellen in peripherem Blut von Patientinnen mit fortgeschrittenem Brustkrebs. Frühere Techniken waren nicht in der Lage eine signifikante Menge zirkulierender Tumorzellen in peripherem Blut zu isolieren, da die Tumorzellen nicht ausreichend angereichert werden konnten. Durch das erfindungsgemäße Verfahren ist es möglich, Tumorzellen in peripherem Blut so stark anzureichern, daß in Kombination mit Detektionsmethoden wie Immunzytochemie, PCR oder Durchflußzytometrie die Identifizierung zirkulierender Tumorzellen möglich wird.

## Patentansprüche

1. Verfahren zum Nachweis von disseminierten Tumorzellen aus einer Körperflüssigkeit, bei dem Nicht-Tumorzellen, die wenigstens eines der Zytokeratine 1-20 exprimieren, von Tumorzellen, die wenigstens eines der Zytokeratine 1-20 exprimieren, abgetrennt werden, worin
(a) Tumorzellen angereichert werden, indem ein Zellseparationsmedium, das eine Dichte im Bereich von 1,055 bis 1,065 g/ml aufweist, mit der Körperflüssigkeit überschichtet wird und zentrifugiert wird, wodurch Zytokeratin-positive von Zytokeratin-negativen Blut-Zellen getrennt werden, wobei sich die angereicherten Tumorzellen in der gleichen Fraktion befinden wie die Zytokeratin-negativen Blut-Zellen; wobei die Zentrifugation in einem Gefäß durchgeführt wird, das durch eine poröse Barriere, einen Filter, ein Sieb oder eine Klappe in ein oberes und ein unteres Kompartiment geteilt ist, wobei das Zellseparationsmedium im unteren Kompartiment vorgelegt wird und die Körperflüssigkeit in das obere Kompartiment eingebracht wird, und wobei die poröse Barriere, der Filter, das Sieb oder die Klappe eine Dicke von 0,5-10 mm, vorzugsweise von 1-5 mm aufweisen; und
(b) bestimmt wird, ob die angereicherten Zellen einen epithelialen Marker exprimieren, der Zytokeratin ist, **dadurch gekennzeichnet, dass** man mRNA aus den angereicherten Zellen revers transkribiert und eine PCR mit wenigstens einem Zytokeratin-spezifischen Primer durchführt, wobei das Zytokeratin ausgewählt ist aus der Gruppe bestehend aus Zytokeratin 1 bis 20.

2. Verfahren nach Anspruch 1, **dadurch gekennzeichnet, dass** die poröse Barriere, der Filter, das Sieb oder die Klappe eine Porengröße von 20-100 µm, vorzugsweise 20-30 µm aufweisen.

3. Verfahren nach einem der Ansprüche 1 oder 2, **dadurch gekennzeichnet, dass** die poröse Barriere, der Filter, das Sieb oder die Klappe aus einem hydrophoben Material bestehen oder mit einem hydrophoben Material beschichtet sind.

4. Verfahren nach einem der vorhergehenden Ansprüche, **dadurch gekennzeichnet, dass** das Zellseparationsmedium einen Farbstoff enthält, der das Zellseparationsmedium von der darüberliegenden Körperflüssigkeit farblich unterscheidbar macht und dadurch die Lokalisation der Interphase vereinfacht.

5. Verfahren nach einem der vorhergehenden Ansprüche, **dadurch gekennzeichnet, dass** in Schritt (b) in Einzel- oder Kombinationsanalyse bestimmt wird, ob die angereicherten Zellen wenigstens einen epithelialen Marker, nämlich eines der Zytokeratine 1-20 exprimieren.

6. Kit enthaltend ein flüssiges Zellseparationsmedium, das eine Dichte im Bereich von 1,055-1,065 g/ml aufweist, wenigstens ein Zentrifugationsgefäß, das durch eine poröse Barriere, einen Filter, ein Sieb oder eine Klappe in ein oberes und ein unteres Kompartiment geteilt ist, wobei das Zellseparationsmedium im unteren Kompartiment vorgelegt wird und die Körperflüssigkeit in das obere Kompartiment eingebracht wird, und wobei die poröse Barriere, der Filter das Sieb oder die Klappe eine Dicke von 0,5-10 mm, vorzugsweise von 1-5 mm aufweisen, und Mittel zum Nachweis der Expression des epithelialen Markers Zytokeratin, nach der Anreicherung direkt oder nach Zellkultur und Expansion der Tumorzellen, **dadurch gekennzeichnet, dass** das Mittel zum Nachweis der Expression von wenigstens einem der Zytokeratine 1-20 ausgewählt ist aus Zytokeratin-spezifischen Primern.

7. Kit nach Anspruch 6, **dadurch gekennzeichnet, dass** weiterhin ein Waschpuffer, gegebenenfalls in konzentrierter Form, zum Waschen der angereicherten Zellen enthalten ist.

## Claims

1. A method for detecting disseminated tumor cells from a body fluid, in which non-tumor cells which express at least one of cytokeratins 1-20 are separated from tumor cells which express at least one of cytokeratins 1-20, wherein
(a) tumor cells are enriched by a cell separation medium which has a density in the range from 1.055 to 1.065 g/ml being overlaid with the body fluid and being centrifuged, wherein cytokeratin-positive and cytokeratin-negative blood cells are separated from one another, with the enriched tumor cells being present in the same fraction as the cytokeratin-negative blood cells; wherein the centrifugation is carried out in a vessel which is divided by a porous barrier, a filter, a sieve or a flap into an upper and a lower compartment, the cell separation medium being introduced into the lower compartment, and the body fluid being put in the upper compartment and wherein the porous barrier, the filter, the sieve or the flap have a thickness of 0.5-10 mm, preferably of 1-5 mm; and
(b) it is determined whether the enriched cells express an epithelial marker, which is cytokeratin, **characterized in that** it comprises reverse transcription of mRNA from the enriched cells and carrying out a PCR with at least one cytokeratin-specific primer, wherein the cytokeratin is selected from the group consisting of cytokeratin 1 to 20.

2. The method as claimed in claim 1, **characterized in that** the porous barrier, the filter, the sieve or the flap have a porous size of 20-100 µm, preferably 20-30 µm.

3. The method as claimed in any of claims 1 or 2, **characterized in that** the porous barrier, the filter, the sieve or the flap consist of a hydrophobic material or are coated with a hydrophobic material.

4. The method as claimed in any of the preceding claims, **characterized in that** the cell separation medium comprises a dye which makes the cell separation medium distinguishable in color from the overlying body fluid, and thus simplifies location of the interphase.

5. The method as claimed in any of the preceding claims, **characterized in that** in step b) there is determination in single or combination analysis of whether the enriched cells express at least one epithelial marker, namely one of cytokeratins 1-20.

6. A kit comprising a fluid cell separation medium which has a density in the range from 1.055-1.065 g/ml, at least one centrifugation vessel, which is divided by a porous barrier, a filter, a sieve or a flap into an upper and a lower compartment, the cell separation medium being introduced into the lower compartment, and the body fluid being put in the upper compartment, wherein the porous barrier, the filter, the sieve or the flap have a thickness of 0.5-10 mm, preferably of 1-5 mm, and means for detecting the expression of the epithelial marker cytokeratin, either directly after the enrichment or after cell culture and expansion of the tumor cells, **characterized in that** the means for detecting the expression of at least one of cytokeratins 1-20 is selected from the group consisting of cytokeratin-specific primers.

7. The kit as claimed in claim 6, **characterized in that** a washing buffer, optionally in concentrated form, is additionally present for washing the enriched cells.

## Revendications

1. Procédé pour détecter des cellules tumorales disséminées, à partir d'un liquide corporel, dans lequel on sépare des cellules non tumorales, qui expriment au moins l'une des cytokératines 1 à 20, des cellules tumorales, qui expriment au moins l'une des cytokératines 1 à 20, dans lequel :
(a) on procède à un enrichissement des cellules tumorales en recouvrant un milieu de séparation cellulaire, qui présente une masse volumique située dans la plage de 1,055 à 1,065 g/ml, avec le liquide corporel, et en soumettant le tout à une centrifugation, ce qui permet de séparer les cellules sanguines positives à la cytokératine des cellules sanguines négatives à la cytokératine, l'enrichissement en cellules tumorales se produisant dans la même fraction que celle contenant les cellules sanguines négatives à la cytokératine ; la centrifugation étant réalisée dans un récipient qui, par le biais d'une barrière poreuse, d'un filtre, d'un tamis ou d'une valve, est divisé en un compartiment supérieur et en un compartiment inférieur, dans lequel on place le milieu de séparation cellulaire dans le compartiment inférieur et on introduit le liquide corporel dans le compartiment supérieur, et dans lequel la barrière poreuse, le filtre, le tamis ou la valve présente une épaisseur de 0,5 à 10 mm, de préférence, de 1 à 5 mm ; et
(b) on détermine si les cellules enrichies expriment un marqueur épithélial, en l'occurrence une cytokératine, **caractérisé en ce que** l'on soumet l'ARNm provenant des cellules enrichies à une transcription inverse et **en ce que** l'on réalise une PCR avec au moins une amorce spécifique à une cytokératine, la cytokératine étant choisie dans le groupe constitué des cytokératines 1 à 20.

2. Procédé selon la revendication 1, **caractérisé en ce que** la barrière poreuse, le filtre, le tamis ou la valve présente une taille de pores de 20 à 100 µm, de préférence, de 20 à 30 µm.

3. Procédé selon l'une quelconque des revendications 1 ou 2, **caractérisé en ce que** la barrière poreuse, le filtre, le tamis ou la valve se compose d'un matériau hydrophobe ou présente un revêtement d'un matériau hydrophobe.

4. Procédé selon l'une quelconque des revendications précédentes, **caractérisé en ce que** le milieu de séparation cellulaire contient un colorant, qui permet de différencier par la couleur le milieu de séparation cellulaire du liquide corporel susjacent, et qui simplifie ainsi la localisation de la phase intermédiaire.

5. Procédé selon l'une quelconque des revendications précédentes, **caractérisé en ce que** l'on détermine à l'étape (b), par une analyse individuelle ou combinée, si les cellules enrichies expriment au moins un marqueur épithélial, à savoir l'une des cytokératines 1 à 20.

6. Trousse contenant un milieu de séparation cellulaire liquide, qui présente une masse volumique située dans la plage de 1,055 à 1,065 g/ml, au moins un récipient de centrifugation, qui est divisé par le biais d'une barrière poreuse, d'un filtre, d'un tamis ou d'une valve, en un compartiment supérieur et en un compartiment inférieur, dans lequel on place le milieu de séparation cellulaire dans le compartiment inférieur et on introduit le liquide corporel dans le compartiment supérieur, et dans lequel la barrière poreuse, le filtre, le tamis ou la valve présente une épaisseur de 0,5 à 10 mm, de préférence, de 1 à 5 mm, et des moyens pour détecter l'expression du marqueur épithélial cytokératine, après l'étape d'enrichissement d'une manière directe, ou après avoir mis en culture et amplifié les cellules tumorales, **caractérisée en ce que** le moyen de détection de l'expression d'au moins l'une des cytokératines 1 à 20 est choisi parmi des amorces spécifiques à une cytokératine.

7. Trousse selon la revendication 6, **caractérisée en ce qu'**elle contient également un tampon de lavage, éventuellement, sous forme concentrée, pour le lavage des cellules enrichies.
